# EUROPÄISCHE PATENTSCHRIFT

(11) **EP 0 226 843 B1**
(45) Veröffentlichungstag und Bekanntmachung des Hinweises auf die Patenterteilung: **28.04.1993**
(21) Anmeldenummer: 86116282.4
(22) Anmeldetag: 24.11.1986
(51) Int. Cl.: G01N 21/47, G01N 33/36, G01B 11/30

(54) **Vorrichtung zur Messung der Haarigkeit eines Garnes**
Device for measuring the hairiness of a yarn
Appareil pour mesurer la pilosité d'un fil

(30) Priorität: 17.12.1985 CH 5370/85
(43) Veröffentlichungstag der Anmeldung: 01.07.1987
(73) Patentinhaber: ZELLWEGER USTER AG, CH-8610 Uster (CH)
(72) Erfinder: Ernst, Felix, CH-8610 Uster (CH); Wampfler, Hans, Dr., CH-8049 Zürich (CH)

(56) Entgegenhaltungen:
- PT-A- 75 791
- TEXTILE PROGRESS, Band 13, Nr. 1, 1983, Seiten 2-31, The Textile Institute, Manchester, GB; A. BARELLA: "Yarn hairiness"

## Beschreibung

Die vorliegende Erfindung betrifft eine Vorrichtung zur Messung der Haarigkeit von Garnen, mit einem Sender zur Beleuchtung des Garns, mit einer Senderlinse, mit einem Empfänger zur Messung der Intensität des durch vom Garn abstehende Fasern verursachten Streulichts, mit einer Empfangsoptik, und mit Mitteln zur Unterdrückung des vom Sender ausgesandten direkten Lichts am Empfänger.

Bei einem in der PT-A-75 791 beschriebenen Verfahren zur Messung der Haarigkeit von Garnen wird als Messprinzip eine Fouriermethode verwendet, mit der am Empfänger die Intensität des durch die vom Garn abstehenden Fasern hervorgerufenen Streulichts gemessen wird. Das direkte Licht wird durch eine im Brennpunkt der Empfangsoptik, zwischen dieser und dem Empfänger, angeordnete kleine Fourierblende unterdrückt.

Da bei dieser Fouriermethode die Lichtausbeute relativ gering ist, muss als Sender ein Laser verwendet werden, was unter Umständen eine gewisse Einschränkung bedeutet. Denn Laserdioden sind heikel zu handhaben und können durch statische Ladungen, Ueberspannungen oder Gegenspannungen zerstört werden. Ausserdem sind Laserdioden teuer. Die Fourierblende muss sehr genau zentriert werden, was einen zusätzlichen Aufwand bedeutet.

Wie praktische Versuche gezeigt haben, ist die in der PT-A-75 791 beschriebene Vorrichtung extrem anfällig auf Verstaubung der optischen Teile. Denn auf einer Linsenoberfläche liegender Staub erzeugt ein gleiches Messsignal wie vom Garnkörper abstehende Haare. Noch gravierender ist der Einfluss durch andere Verschmutzungen, die das Messergebnis um eine Mehrfaches verfälschen können.

Durch die Erfindung soll nun eine Vorrichtung der eingangs genannten Art angegeben werden, bei der die Lichtausbeute so gross ist, dass als Sender nicht unbedingt ein Laser verwendet werden muss, und bei der die für die Unterdrückung des vom Sender ausgesandten direkten Lichts erforderliche Blende mit möglichst geringem Aufwand eingestellt werden kann. Ausserdem soll die Vorrichtung unempfindlich gegen Staub und Verschmutzungen sein.

Diese Aufgabe wird erfindungsgemäss dadurch gelöst, dass die genannten Mittel durch eine im Strahlengang vor der Empfangsoptik angeordnete zentrale Blende gebildet sind, und dass zwischen der Empfangsoptik und dem Empfänger eine Absorberblende zur Unterdrückung von durch im Strahlengang angeordnete optische Elemente verursachtem Streulicht vorgesehen ist.

Die erfindungsgemässe Vorrichtung basiert nicht auf einer Fouriermethode, sondern auf einer Dunkelfeldmessmethode, deren Lichtausbeute so gross ist, dass als Sender auch eine Infrarot-Leuchtdiode verwendet werden kann.

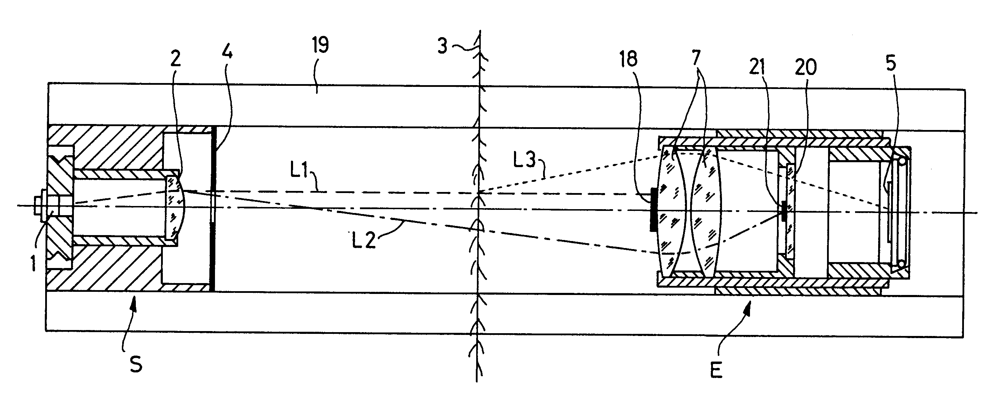

Nachstehend wird die Erfindung anhand eines in der einzigen Zeichnung dargestellten Ausführungsbeispiels näher erläutert.

Die dargestellte Messvorrichtung für die Haarigkeit von Garnen besteht aus zwei Baugruppen, einem Senderteil S und einem Empfängerteil E, welche auf einer Grundplatte 19 montiert sind. Der Senderteil S enthält neben einer als Sender dienenden Leuchtdiode 1 eine Kondensorlinse 2, die das Licht der Leuchtdiode 1 in ein paralleles Strahlenbündel zusammenfasst, sowie eine Messfeldblende 4. Der Empfängerteil E enthält eine Empfangsoptik 7 mit einer zentralen Blende 18 und mit einem Empfänger 5. Zwischen Empfangsoptik 7 und Empfänger 5 ist ein Glasträger 20 mit einer Absorberblende 21 angeordnet.

Wesentlich für das bei der Messvorrichtung angewandte Messverfahren ist die Auswertung des vom Garn 3 durch diffuse Streuung, Brechung, Beugung und/oder Reflexion ausgesandten Lichtes mit Hilfe einer sogenannten Dunkelfeldoptik. Dies bedeutet mit anderen Worten, dass vom Empfänger 5 die vom Sender 1 beleuchtete Seite des Garns 3 nicht eingesehen wird, so dass also der Empfänger 5 nur Licht von der unbeleuchteten Seite des Garns 3 erhält.

Das vom Empfänger 5 empfangene Licht ist damit unabhängig vom Garndurchmesser und das von der beleuchteten Oberfläche des Garns 3 diffus zurückreflektierte Licht kann nicht auf den Empfänger 5 treffen, so dass dieser nur den hellen Garnrand sieht. Im Empfänger 5 entsteht ein kontinuierliches Analogsignal, so dass die Haarigkeit des Garns 3 kontinuierlich aufgezeichnet werden kann. Denn auf den Empfänger 5 gelangt nur das an den vom Rand des Garnes 3 abstehenden Fasern - die für die Haarigkeit verantwortlich sind - diffus gestreute, reflektierte und gebrochene Streulicht und dessen Intensität ist, so wie im portugiesischen Patent Nr. 75 791 beschrieben, proportional zur Anzahl der abstehenden Fasern und somit zur Haarigkeit des Garnes.

Als Sender 1 wird vorzugsweise eine Infrarot-Leuchtdiode (IRED) verwendet, deren Licht vorzugsweise moduliert ist, damit im Empfängerpfad das Umgebungslicht, beispielsweise mit einem Bandpassfilter, herausgefiltert werden kann. Es ist ausserdem vorteilhaft, das Umgebungslicht etwas abzuschirmen. Selbstverständlich könnte als Sender 1 auch eine sichtbare LED, ein Laser oder irgendeine andere Lichtquelle verwendet werden.

Der Empfänger 5 ist entweder eine grossflächige Fotodiode, um möglichst viel Streulicht zu empfangen und um möglichst unabhängig von durch Lageveränderungen und Vibrationen des Garnes verursachten Verschiebungen der Garnabbildung zu sein, oder ein Fotomultiplier, oder eine CCD-Reihenzelle, auf welche das Garn abgebildet wird. Im letzteren Fall kann man die Verteilung der Haarigkeit im Abstand vom Garnrand messen und den Durchmesser des Garnes 3 bestimmen.

Die Auswertung der Signale des Empfängers 5 wird als für den Durchschnittsfachmann bekannt vorausgesetzt und ist deswegen im vorliegenden Patent nicht näher erläutert. Es wird in diesem Zusammenhang auf die schon eingangs erwähnte PT-A-75 791 verwiesen sowie auf die Broschüre "Yarn Hairiness", Textile Progress, Volume 13, No. 1, The Textile Institute, Manchester.

Die Vorrichtung zur Messung der Haarigkeit enthält selbstverständlich auch einen mechanischen Teil für das Einlegen und den Abzug des zu untersuchenden Garns. Da dieser mechanische Teil prinzipiell gleich ist wie bei heute käuflich erhältlichen Haarigkeitsmessgeräten, die übrigens grösstenteils auf einer Methode zur Zählung der vom Garn abstehenden Fasern beruhen, ist er ebenfalls nicht beschrieben und es wird in diesem Zusammenhang auf die schon genannte Broschüre "Yarn Hairiness" verwiesen.

Das von der Leuchtdiode 1 ausgesandte Strahlenbündel L1, welches durch die Messfeldblende 4 begrenzt wird, wird von der zentralen Blende 18 abgedeckt. Von allenfalls auf der Kondensorlinse 2 liegendem Staub gestreutes Licht gelangt als Lichtstrahl L2 in den Empfängerteil E und geht ausserhalb von der zentralen Blende 18 durch die Empfangsoptik 7, wird aber durch die Absorberblende 21 abgedeckt. So trifft also letztlich nur das am Garn 3 gestreute Licht L3 auf den Empfänger 5.

Da bei Messungen an Garnen viel Staub entsteht, sind bei der dargestellten Messvorrichtung verschiedene Massnahmen getroffen, um den Einfluss von Verstaubung auf die Messung zu verringern:
- Der Abstand von Kondensorlinse 2 und Empfangsoptik 7 zum Garn 3 ist gross.
- Die zentrale Blende 18 verhindert, dass Glasteile der Empfangsoptik 7 im direkten Strahlengang liegen. Dadurch wird an Staubteilchen auf der Empfangsoptik 7 kein Streulicht erzeugt.
- Die Kondensorlinse 2 wird durch die Empfangsoptik 7 auf die Absorberblende 21 abgebildet; dadurch wird auf der Kondensorlinse 2 eventuell entstehendes Streulicht abgedeckt und kann nicht auf den Empfänger 5 gelangen.

Die dargestellte Messvorrichtung wird im praktischen Einsatz in ein Gerät eingebaut, welches unter anderem Führungs- und Transportmittel für das Garn aufweist; siehe dazu beispielsweise die CH-A-671 105. In Laufrichtung des Garnes 3, das ist in der Figur von oben nach unten, ist vor dem Messorgan, also in der Figur oben, eine Fadenbremse angeordnet. Im Bereich des Messorgans, kurz vor und nach dem Strahlengang, sind zwei Fadenführungen vorgesehen, und unterhalb des Messorgans sind zwei Abzugsrollen für das Garn 3 angeordnet. Die Abzugsgeschwindigkeit beträgt bis zu mehreren Metern pro Sekunde.

Um störende Einflüsse durch das Umgebungslicht auszuschliessen, wird der Sender 1 moduliert. Der im Empfänger 5 erzeugte Fotostrom wird in eine proportionale Spannung umgewandelt und dieses Signal wird verstärkt, gleichgerichtet, durch einen Tiefpass gefiltert und anschliessend durch einen Rechner ausgewertet. Dabei ist die über die Zeit gemittelte Spannung ein Mass für die mittlere Haarigkeit. Es lassen sich aber auch kurzzeitige Schwankungen der Haarigkeit oder, mittels Fouriertransformation, Perioden der Haarigkeit erfassen.

Zur Kompensation einer eventuellen Temperaturdrift von Sender und/oder Empfänger oder von Alterungseinflüssen kann im Strahlengang des Senderteils 8 bzw. des Empfängerteils E eine Referenzdiode vorgesehen sein. Diese ermöglicht bei aus der Vorrichtung entferntem Garn 3 die Feststellung der genannten Drift und Einflüsse und damit auch deren Kompensation. Wenn die Referenzdiode im Empfängerteil E angeordnet ist, was einen zusätzlichen Referenzpfad vom Sender zur Referenzdiode erfordert, dann kann in diesem Referenzpfad der Garndurchmesser bestimmt werden.

Zur Verhinderung von Verschmutzung können die optischen Elemente in Ueberdruckbehältern angeordnet sein, deren ausströmende Luft das Eindringen von Staub und Schmutz verhindert, oder es können entsprechende Luftvorhänge verwendet werden.

## Patentansprüche

1. Vorrichtung zur Messung der Haarigkeit von Garnen mit einem Sender (1) zur Beleuchtung des Garns (3), mit einer Senderlinse (2), mit einem Empfänger (5) zur Messung der Intensität des durch vom Garn abstehende Fasern verursachten Streulichts (L3), mit einer Empfangsoptik (7), und mit Mitteln zur Unterdrückung des vom Sender ausgesandten direkten Lichts (L1) am Empfänger, dadurch gekennzeichnet, dass die genannten Mittel durch eine im Strahlengang vor der Empfangsoptik (7) angeordnete zentrale Blende (18) gebildet sind, und dass zwischen der Empfangsoptik und dem Empfänger (5) eine Absorberblende (21) zur Unterdrückung von durch im Strahlengang angeordnete optische Elemente verursachtem Streulicht (L2) vorgesehen ist.

2. Vorrichtung nach Anspruch 1, dadurch gekennzeichnet, dass durch die Empfangsoptik (7) eine Abbildung der Senderlinse (2) auf die Absorberblende (21) erfolgt.

3. Vorrichtung nach Anspruch 2, dadurch gekennzeichnet, dass eine Messfeldblende (4) zur Festlegung des Messfelds auf dem Empfänger (5) vorgesehen ist, und dass der Abstand zwischen der Senderlinse (2) und dem Garn (3) einerseits und dem Garn und der Empfangsoptik (7) andererseits ein Mehrfaches des Durchmessers des genannten Messfeldes beträgt.

4. Vorrichtung nach Anspruch 2, dadurch gekennzeichnet, dass der Sender (1) durch eine Infrarot-Leuchtdiode gebildet ist.

5. Vorrichtung nach Anspruch 4, dadurch gekennzeichnet, dass das vom Sender (1) ausgestrahlte Licht moduliert ist.

6. Vorichtung nach Anspruch 2, dadurch gekennzeichnet, dass der Empfänger (5) durch ein fotoelektrisches Element in der Art einer Fotodiode, eines Fotovervielfachers oder eines ladungsgekoppelten Bauelements (CCD) gebildet ist

7. Vorrichtung nach einem der Ansprüche 1 bis 6, dadurch gekennzeichnet, dass der Sender (1) und/oder der Empfänger (5) gegen Verstaubung und/oder Verschmutzung geschützt ist, vorzugsweise durch Anordnung entsprechender Luftvorhänge oder durch Einbau in Gehäuse.

8. Vorrichtung nach Anspruch 7, dadurch gekennzeichnet, dass das Gehäuse einen leichten Ueberdruck aufweist.

9. Vorrichtung nach einem der Ansprüche 1 bis 6, gekennzeichnet durch einen Referenzempfänger zur Kompensation der Temperaturabhängigkeit und/oder Alterung der Vorrichtung und/oder einer eventuellen, durch Verschmutzung innerhalb der Vorrichtung bedingten Verminderung der Lichtintensität am Empfänger (5).

## Claims

1. Apparatus for measuring the hairiness of yarns, having a transmitter (1) for illuminating the yarn (3), a transmitter lens (2), a detector (5) for measuring the intensity of scattered light (L3) produced by fibres standing up from the yarn, with a receiving optical system (7), and with means for suppressing at the receiver the direct light (L1) emitted by the transmitter, characterized in that the said means are formed by a central diaphragm (18) being arranged in the optical path in front of the receiving optical system (7), and in that an absorption diaphragm (21) is provided between the receiving optical system and the detector (5) for suppressing the scattered light (L2) produced by optical elements arranged in the optical path.

2. Apparatus according to claim 1, characterized in that the receiving optical system (7) produces a mapping of the transmitter lens (2) on the absorption diaphragm (21).

3. Apparatus according to claim 2, characterized in that a measuring field diaphragm (4) is provided for determining the measuring field on the detector (5), and in that the distance between the transmitter lens (2) and the yarn (3) on the one hand, and between the yarn and the receiving optical systems (7) on the other hand amounts to a multiple of the diameter of the said measuring field.

4. Apparatus according to claim 2, characterized in that the transmitter (1) is formed by an infrared light-emitting diode.

5. Apparatus according to claim 4, characterized in that the light emitted by the transmitter (1) is modulated.

6. Apparatus according to claim 2, characterized in that the detector (5) is formed by a photoelectric element in the manner of a photodiode, a photomultiplier or a charge-coupled device (CCD).

7. Apparatus according to one of claims 1 to 6, characterized in that the transmitter (1) and/or the detector (5) is protected against dust and/or dirt collection, preferably by providing suitable air curtains or by mounting in casings.

8. Apparatus according to claim 7, characterized in that the casing has a slight overpressure.

9. Apparatus according to one of claims 1 to 6, characterized by a reference detector to compensate for the temperature drift and/or ageing of the device and/or a possible reduction of the light intensity at the detector (5) due to dirt collection within the device.

## Revendications

1. Dispositif pour la mesure de la pilosité de fils avec un émetteur (1) pour éclairer le fil (3), avec une lentille émettrice (2), avec un récepteur (5) pour mesurer l'intensité de la lumière diffusée (L3) provoquée par des fibres faisant saillie du fil, avec une optique de réception (7) et avec des moyens pour supprimer la lumière directe (L1), émise par l'émetteur, au récepteur, caractérisé en ce que les moyens précités sont constitués par un obturateur ou diaphragme central (18) disposé dans le trajet des rayons devant l'optique de réception (7), et en ce qu'il est prévu entre l'optique de réception et le récepteur (5) un diaphragme d'absorption (21) pour supprimer la lumière diffusée (L2) provoquée par des éléments optiques disposés dans le trajet des rayons.

2. Dispositif selon la revendication 1, caractérisé en ce que, du fait de l'optique de réception (7), il se produit une représentation de la lentille émettrice (2) sur le diaphragme d'absorption (21).

3. Dispositif selon la revendication 2, caractérisé en ce qu'un diaphragme du champ de mesure (4) est prévu pour déterminer le champ de mesure sur le récepteur (5), et en ce que l'écart entre la lentille émettrice (2) et le fil (3) d'une part, et le fil et l'optique de réception (7) d'autre part constituent un multiple du diamètre dudit champ de mesure.

4. Dispositif selon la revendication 2, caractérisé en ce que l'émetteur (1) est constitué par une diode luminescente infrarouge.

5. Dispositif selon la revendication 4, caractérisé en ce que la lumière rayonnée par l'émetteur (1) est modulée.

6. Dispositif selon la revendication 2, caractérisé en ce que le récepteur (5) est constitué par un élément photoélectrique du type à photodiode, à photomultiplicateur ou à dispositif à couplage de charge (CCD).

7. Dispositif selon l'une des revendications 1 à 6, caractérisé en ce que l'émetteur (1) et/ou le récepteur (5) sont protégés contre la poussière et/ou l'encrassement, de préférence par la disposition de rideaux d'air correspondants ou par insertion dans des boîtiers.

8. Dispositif selon la revendication 7, caractérisé en ce que le boîtier présente une légère surpression.

9. Dispositif selon l'une des revendications 1 à 6, caractérisé par un récepteur de référence pour compenser la dépendance de température et/ou le vieillissement du dispositif et/ou une diminution éventuelle de l'intensité de lumière au récepteur (5) provenant d'un encrassement à l'intérieur du dispositif.
